# EUROPEAN PATENT APPLICATION

(11) **EP 4 563 098 A2**
(43) Date of publication of application: **04.06.2025**
(21) Application number: 25151075.6
(22) Date of filing: 11.04.2019
(51) Int. Cl.: A61B 17/12

(54) **VASCULAR IMPLANT WITH ANCHOR MEMBER**

(30) Priority: 11.04.2018 EP 18166718
(62) Divisional of application: 19718638.0
(71) Applicant: Occlutech GmbH, 07745 Jena (DE)
(72) Inventor: MORGAN, Ross, 07743 Jena (DE); GROSSHAUSER, Johannes, 07743 Jena (DE)
(74) Representative: KIPA AB

(57) **Abstract**

An expandable vascular implant is disclosed, which comprises a framework with openings and forming an exterior surface of the implant, and at least one anchor member for anchoring the implant to a vascular structure and having a proximal portion and a distal portion. The distal portion of the anchor member comprises at least one free end configured to extend outwardly from the framework. The anchor member is at least partly interlaced with the framework between the proximal portion and the distal portion such that the anchor member is fixed to the framework.

## Description

### Field of the Invention

The invention relates to an expandable vascular implant, such as an occluder for occluding a vascular defect. The implant comprises a framework to which at least one anchor member for anchoring the implant to a vascular structure is attached. The anchor member is at least partly interlaced with the framework, such as between a proximal portion and a distal portion of the anchor member, such that the anchor member is fixed to the framework.

The invention also relates to such an anchor member, which may be provided separately from the implant.

### Background of the Invention

An occluder is a medical product or implant used for occluding defects, e.g. in the human heart. Defects may occur in various regions of the heart and have different forms. Defects in the septum of the atrium are common.

The occluders can be inserted using minimally invasive cardiac catheter techniques, more precisely by means of a transvenous, catheter-interventional access.

Being projections from the atria, auricles are parts of the heart and not defects.

In the case of patients who are susceptible to atrial fibrillation or suffering from arrhythmia, the auricle may be the origin of blood clots. Thus, occluding the left auricle can prevent the creation of thrombi and reduce the risk of a stroke.

There are some left atrial appendage (LAA) occluders known for this purpose. However, it may be difficult to make the LAA occluders stay in the right position once implanted.

The LAA occluder in US2011054515 solves this by the use of barbs that are formed integral with a frame, i.e. the frame including barbs is monolithic. The frame is covered by a membrane that is attached to a portion of the frame. The membrane can be attached to the frame with stitching, hooks, tangs or stakes. However, the use of a membrane is not favourable in all situations.

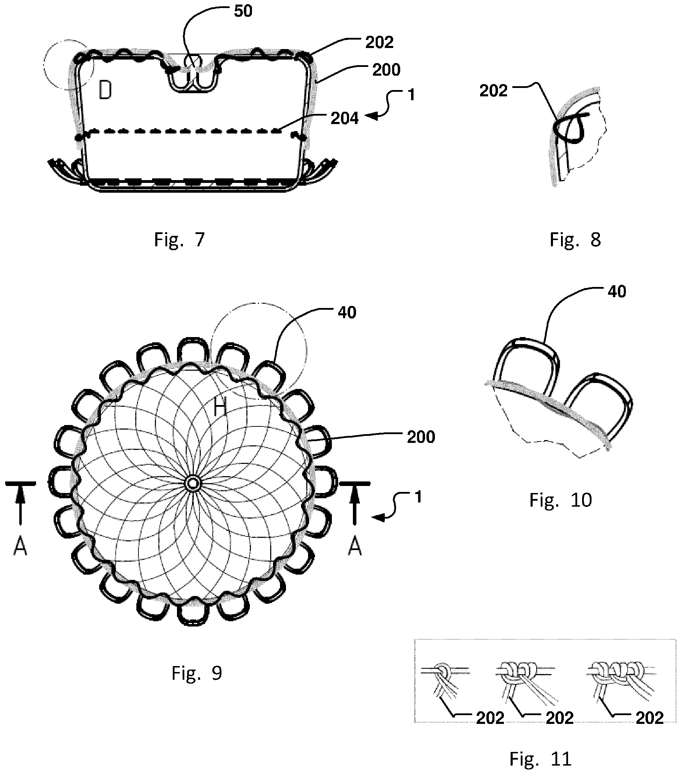

Another LAA occluder is known from US8758389. In this document, the occluder comprises a braided structure. The positioning of the occluder is secured by the use of hooks. The hooks are sutured or woven to the braided structure, as is illustrated by a zig-zag structure in Fig. 10 of the document.

However, the use of stitching, suturing, weaving, etc. of hooks or barbs, or a membrane to a frame with such hooks or barbs, may be unreliable. The suture, thread, etc. used for the attachment may become loose, with the associated risk of the hooks or barbs dislodging from the other structure, and the occluder losing its position relative the defect or structure to which it is attached. There is also the risk that the hooks or bars damage the catheter when the implant is collapsed and positioned in the catheter for delivery to the target site.

Hence, an improved occluder, which upon implantation may be reliably anchored to a vascular structure, is desired.

### Summary of the Invention

Accordingly, embodiments of the present invention preferably seek to mitigate, alleviate or eliminate one or more deficiencies, disadvantages or issues in the art, such as the above-identified, singly or in any combination by providing a vascular expandable implant with an anchor member, a method for attaching the anchor member to the implant, and at least one anchor member.

The invention is defined by the appended claims.

An embodiment comprises an expandable vascular implant, which comprises a framework, at least one anchor member for anchoring the implant to a vascular structure and having a proximal portion and a distal portion. The distal portion of the anchor member comprises at least one free end configured to extend outwardly from the framework. The anchor member is at least partly interlaced with the framework of the implant, such as between the proximal portion and the distal portion, such that the anchor member is fixed to the framework.

The anchor member may comprise an intermediate section between the proximal portion and the distal portion. The intermediate section may be interlaced with the framework, such as in the longitudinal direction of the implant. The intermediate section may be curved, such as in the longitudinal direction of the implant when attached to the framework. It may be curved such that one part of the intermediate section is located closer to the center of the implant compared to a second part. Hence, a proximal part of the intermediate section may be configured to extend along a first side of the framework. A distal part of the intermediate section may be configured to extend along a second side of the framework. The first side and the second side of the framework may be opposite sides of the framework. Hence, the intermediate section may be curved to follow the profile of each side of the framework. A curve of the intermediate section may extend through an opening of the framework such that the first and second parts conform to the profile or shape of each side of the framework.

The anchor member may comprise an anchor strand formed into the anchor member. A proximal end of the anchor strand may form at least one loop or knot extending around a portion of the framework. The anchor strand may comprise two free ends forming a distal end of the anchor member. The loop may be positioned between the two free ends, such as centered therebetween.

The proximal end of the anchor strand may form two loops that extend around the portion of the framework. A first loop and a second loop of the two loops may extend through the same openings of the framework.

The anchor strand may comprise a first distal end portion and a second distal end portion. At least one loop or bend may be located between the first distal end portion and the second distal end portion. The first distal end portion and the second distal end portion may a form a hook with a free end directed proximally of the implant. The hook may comprise a barb.

The anchor member may be monolithic. Also, the anchor member may comprise a plurality of interconnected bars. At least one of the plurality of bars may comprise a cross-bar. The cross-bar may be interlaced with the framework in a radial or longitudinal direction of the implant.

The interconnected bars may form a circumference of the anchor member. The circumference may form a plurality of peaks with a valley between adjacent peaks of the plurality of peaks. An anchor may be located at a peak and/or a valley of the circumference. The anchor may comprise a hook, which may comprise a barb. The anchor member may comprise a plurality of fixation tabs spaced around the circumference. The fixation tabs may extend in the longitudinal direction of the implant and on opposite sides of the framework.

Bars of the anchor member may extend between the peaks and the valley and on an inner side of the framework. At least one of the proximal portion and the distal portion of the anchor member may extend from at least one of the peak and the valley and on an exterior side of the framework.

The anchor member and the implant may be separate components. Hence, in some embodiments, the anchor member disclosed herein is provided as a separate component and may form an independent embodiment.

Embodiments comprise a method of attaching at least one anchor member to an expandable vascular implant. The method comprises providing an expandable vascular implant comprising a framework; providing at least one anchor member having a proximal portion and a distal portion; and interlacing at least a portion of the anchor member, such as between the proximal portion and the distal portion, with the framework. The method may comprise attaching an anchor member such that at least one free end of the anchor member, such as at the distal portion or the proximal portion of the anchor member, extends outwardly from the framework.

Providing the anchor member may comprise providing the anchor member in a shape memory material that has been pre-treated to its relaxed shape. Interlacing the anchor member with the framework may comprise interlacing the anchor member, made of the pre-treated shape memory material, with said framework.

Providing the anchor member may comprise providing the anchor member as an anchor strand. Interlacing at least a portion of the anchor member with the framework may comprise interlacing at least a portion of the anchor strand and forming at least one loop or knot with the anchor strand around a portion of the framework.

Some embodiments of the invention provide for an expandable vascular implant that provides for stability of the implant when attached to the vascular structure. Particularly, the anchor member, which may be interlaced with the framework, can be reliably attached to the framework, which provides for the stability, particularly prevents the anchor member from loosening from the framework. Furthermore, the anchor member may have a slim profile in a collapsed configuration, such that damaging a catheter during delivery is prevented. This also contributes to the safety of the implant and delivery of the implant. Delivery of the anchor member as a separate component provides for flexibility during production at the same time as the design of the anchor member may be optimised separately from the design of the framework, which also may optimize production processes.

The term "comprises/comprising" when used in this specification is taken to specify the presence of stated features, integers, steps or components but does not preclude the presence or addition of one or more other features, integers, steps, components or groups thereof.

### Brief Description of the Drawings

These and other aspects, features and advantages of which embodiments of the invention are capable of, will be apparent and elucidated from the following description of embodiments of the present invention, reference being made to the accompanying drawings, in which
Fig. 1a is a side view of an implant with anchor members attached to the framework;
Fig. 1b is a perspective view of an anchor member;
Fig. 1c is a schematic view illustrating attaching the anchoring member of Fig. 1b to the framework;
Fig. 1d is a front view of the anchor member of Fig. 1b attached to the framework;
Fig. 2a is a side view of a vascular expandable implant with an anchor member attached to the framework;
Fig. 2b is a perspective view of an anchor member;
Fig. 2c is a side view of a portion of an anchor member attached to the framework at its distal end;
Fig. 2d is a side view of a portion of an anchor member attached to the framework at its proximal end;
Fig. 3 is a side view of an implant without any anchor member attached to the framework;
Fig. 4 is a top view of an implant;
Figs. 5-6 are side and bottom views of an implant;
Fig. 7 is a cross-sectional view of an implant with a membrane taken along section A-A in Fig. 9;
Fig. 8 is a sectional-view of a thread used for attaching the membrane to the implant;
Fig. 9 is a top view of an implant with strand loops;
Fig. 10 is a sectional-view of the strand loops of Fig. 9;
Fig. 11 is a schematic view of different knots used for attaching the membrane to the implant;
Fig. 12 is a cross-sectional view of an implant with a coupling or attachment member;
Fig. 13 is a cross-sectional view of an implant with a membrane;
Fig. 14 is a view of a thread used for attaching the membrane to the implant;
Fig. 15 is a sectional-view of triangular strand loops of an implant;
Fig. 16 is a top view of the implant with triangular strand loops;
Fig. 17 is a schematic view of different knots for an implant;
Fig. 18 is a cross-sectional view of an implant with a coupling or attachment member;
Fig. 19 is a detailed view of the coupling or attachment member of the implant;
Fig. 20 is a side view of an implant being manufactured;
Fig. 21 is a side view of an implant being manufactured;
Fig. 22a is a side view of an implant with a concave shape;
Fig. 22b is a cross-sectional view of an implant with an outer membrane;

### Description of embodiments

Specific embodiments of the invention now will be described with reference to the accompanying drawings. This invention may, however, be embodied in many different forms and should not be construed as limited to the embodiments set forth herein; rather, these embodiments are provided so that this disclosure will be thorough and complete, and will fully convey the scope of the invention to those skilled in the art. The terminology used in the detailed description of the embodiments illustrated in the accompanying drawings is not intended to be limiting of the invention. In the drawings, like numbers refer to like elements.

The present description of the current invention is given with reference to an occluder for a defect in the heart as an example only. **It** should be born in mind, however, that the present invention is not limited strictly to an occluder but can be easily adapted to other expandable vascular implants, such as a valve replacement stent, or a vascular stent, to be anchored in a vascular structure of the vascular system of a patient. The expandable vascular implant may be delivered via a catheter and a delivery wire in a collapsed configuration, and be expanded to a pre-set or relaxed configuration at the target side. Once deployed, the expandable vascular implant should remain at the target site. The invention is particularly useful for vascular structures that move with the contraction and expansion of the vascular system, such as with heart movements. For example, the invention is particular useful for a left auricular appendix (LAA) occluder. Such occluders have a tendency to be pushed out of the LAA unless the occluder is anchored in the vascular structure as the heart contracts and expands. The present invention provides for reliable anchoring in vascular structures while being collapsible to a small diameter for delivery in a catheter and expansion at the target site. In the following reference will be made to an implant when referring to the expandable vascular implant.

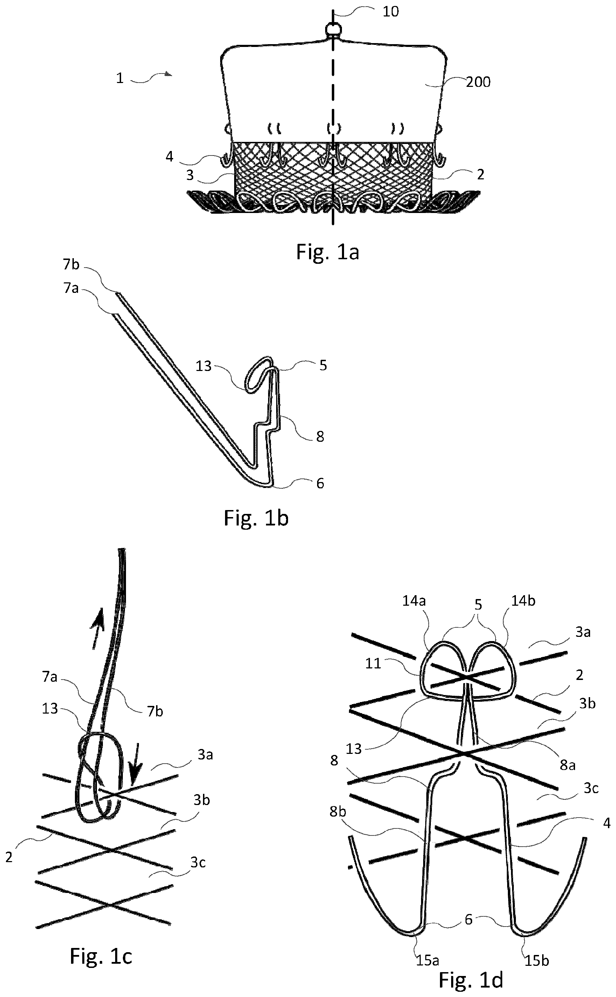

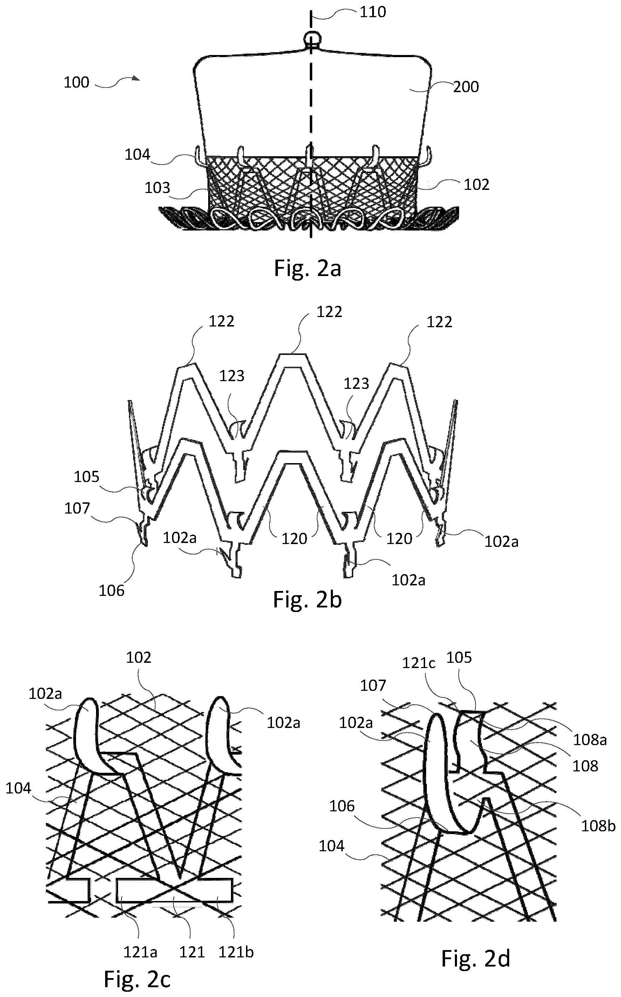

Figs. 1a and 2a illustrate embodiments of the implant 1, 100, which comprises a framework 2, 102 with openings 3, 103. The framework may form an exterior surface of the implant 1, 100. At least one anchor member 4, 104 is attached to the framework 2, 102, and is configured to anchor the implant 1, 100 to the vascular structure.

Fig. 1b illustrates aspects of the anchor member 4 of the embodiment of Fig. 1a. Fig. 1c illustrates attaching the anchor member of Fig. 1b to the framework 2 of the embodiment of Fig. 1a. Fig. 1d illustrates the anchor member 4 attached to the framework 2.

Similarly, Fig. 2b illustrates aspects of the anchor member 104 of the embodiment of Fig. 2a. Fig. 2c-2d illustrate embodiments of the anchor member 104 attached to the framework 102.

As illustrated in Figs. 1b and 2b, the anchor member 4, 104 has a proximal portion 5, 105 and a distal portion 6, 106. The distal portion 6, 106 comprises at least one free end 7, 107 configured to extend outwardly from the framework 2, 102.

As illustrated in Figs. 1d and 2c-2d, the anchor member 4, 104 is at least partly interlaced with the framework 2, 102 between the proximal portion 5, 105 and the distal portion 6, 106 such that the anchor member 4, 104 is fixed or attached to the framework 2, 102. In this context, proximal is a position located closer to an end of the implant 1, 100, to which a delivery device, such as a delivery wire may be attached to the implant. Similarly, distal is a position located closer to a location located further away from the point of attachment of the delivery device to the implant 1, 100 than the proximal position. Interlaced in this context means that a portion of the anchor member 4, 104 extends alternatively on opposite sides of the framework 2, 102, such as on a side facing inwardly towards the center of the implant 1, 100 and a side facing outwardly away from the implant 1, 100. The interlacing may be in the longitudinal direction of the implant. The framework 2, 102 may form boundaries of the openings 3, 103. A portion of the anchor member 4, 104 may extend through multiple openings 3, 103 such that the portion extends on at least one of the opposing sides of the framework 2, 102 between the openings 3, 103 through which the portion of the anchor member 4, 104 extends, such as is illustrated in detail in the embodiments of Figs. 1d, 2c, and 2d.

In some embodiments, the framework 2, 102 comprises a mesh formed by at least one strand. The mesh may be formed by weaving and/or braiding. The mesh may also be formed by a monolithic structure, such as from a hollow tube in which the openings have been formed, e.g., by laser cutting. Examples of strands are Nitinol wires, which may be heat set to a pre-defined shape. Such strands are super-elastic, such that the implant 1, 100 is collapsible for delivery though a catheter, and self-expandable when unconstrained to the relaxed shape, as illustrated in Figs. 1a, and 2a, respectively.

As is illustrated in Figs. 1b and 2d, embodiments of the anchor member 4, 104 comprises an intermediate section 8, 108 that may be located between the proximal portion 5, 105 and the distal portion 107. The intermediate section 8, 108 may be interlaced with the framework 2, 102, as is illustrated in Figs. 1d and 2d. As can be seen in these figures, a first part 8a, 108a, which may be a proximal part, of the intermediate section 8, 108 may be located on a first side of the framework 2, 102, and a second part 8b, 108b, which may be a distal part, of the intermediate section 8, 108 may be located on a second side of the framework 2, 102. The first side and the second side may be opposing sides of the framework 2, 102, such as an inner side and an outer side of the framework 2, 102. The first part 8a, 108a, may be distal of the second part 8b, 108b, or vice versa. The first part 8a, 108a, and the second part 8, 108b may be radially aligned but axially spaced apart relative a longitudinal axis 10, 110 of the implant 1, 100. Hence, the intermediate section 8, 108 may be interlaced with the framework 2, 102 in the longitudinal direction of the implant 1, 100. Thus, the anchor member 4, 104 may be supported by the framework 2, 102 in the radial as well as the axial direction. This provides for reliably anchoring of the implant 1, 100 to the vascular structure.

As can be seen in Figs. 1b and 2b, the intermediate section 8, 108 may be curved, such as in its longitudinal direction when mounted in the longitudinal direction of the framework 2, 102. The first part 8a, 108a, of the intermediate section 8, 108 may be configured to extend along a first side of said framework 2, 102, and the second part 8b, 108b of the intermediate section 8, 108 may be configured to extend along the second side of the framework 2, 102. Again, the first side and the second side of the framework 2, 102 may be opposite sides of the framework 2, 102. The first part 8a, 108a may be curved to be located closer to the centre of the implant 1, 100 than the second part 8b, 108b when fixed to the framework 2, 102. The second part 8b, 108b may be curved to be located further away from the centre of the implant 1, 100 than the first part 8a, 108a when fixed to the framework 2, 102. A bend or curve may be located between the first part 8a, 108a, and the second part 18b, 108b. The bend or curve may be positioned at an opening of the framework 2, 102. Hence, the anchor member 4, 104 is configured to substantially not affect the relaxed shape of the framework 2, 102. At the same time, the anchor member 4, 104 may extend along the inner and outer surface of the framework 2, 102, and may even abut these surfaces even when the implant is in the relaxed or expanded shape. Hence, the profile of the anchor member 4, 104, may closely follow the profile of the framework 2, 102. When collapsed for delivery, the implant will assume a slim profile, which facilitates using a catheter with a relatively small diameter. Also, the curved profile of the anchor member 4, 104 avoids damaging the inside of the catheter, which prevents particles from a damaged catheter entering into the vascular system during delivery. Hence, the curved profile may also enhance the safety of the implant 1, 100.

Figs. 1a-1d illustrate embodiments of the anchor member 4 comprising an anchor strand formed into the anchor member 4. The anchor strand may be made of a shape memory material, such as a shape memory metal, e.g. Nitinol. The anchor strand may be heat or otherwise treated to assume a pre-configured shape with the proximal portion 5, the distal portion 6, and the intermediate section 8. Also, the anchor strand may be heat treated separately from the framework 2 before it is attached to the framework 2. The anchor member 4 may be double sided, i.e. have mirrored sides having the same configuration between the free end 7 to a centre of the strand, which is located in the middle between the free ends of the strand.

As is illustrated in Fig. 1d, when the anchor strand is attached to the framework 2, a proximal end 11 of the anchor strand may form at least one loop or knot extending around a portion of the framework 2. The anchor strand may comprise two free ends 7 forming a distal end of the anchor member 4. The loop(s) or knot may be positioned between the two free ends, such as centred between the free ends 7. Hence the anchor strand may extend in the distal direction from a first free end 7a towards the distal end 6 of the anchor member 4: in the proximal direction from the distal end 6 of the anchor member 4 towards the proximal end 5 of the anchor member 4 and interlaced with the framework 2; form at least one loop or knot around the framework 2; in the distal direction from the proximal end 5 of the anchor member 4 towards the distal end 6 of the anchor member 4 and interlaced with the framework 2; and in the proximal direction from the distal end 6 of the anchor member 4 to a second free end 7b. In other embodiments, the second free end 7b of the anchor strand ends at the proximal end of the anchor member 4 after a loop or knot has been formed. Hence, the anchor strand may form one or two legs of the anchor member 4.

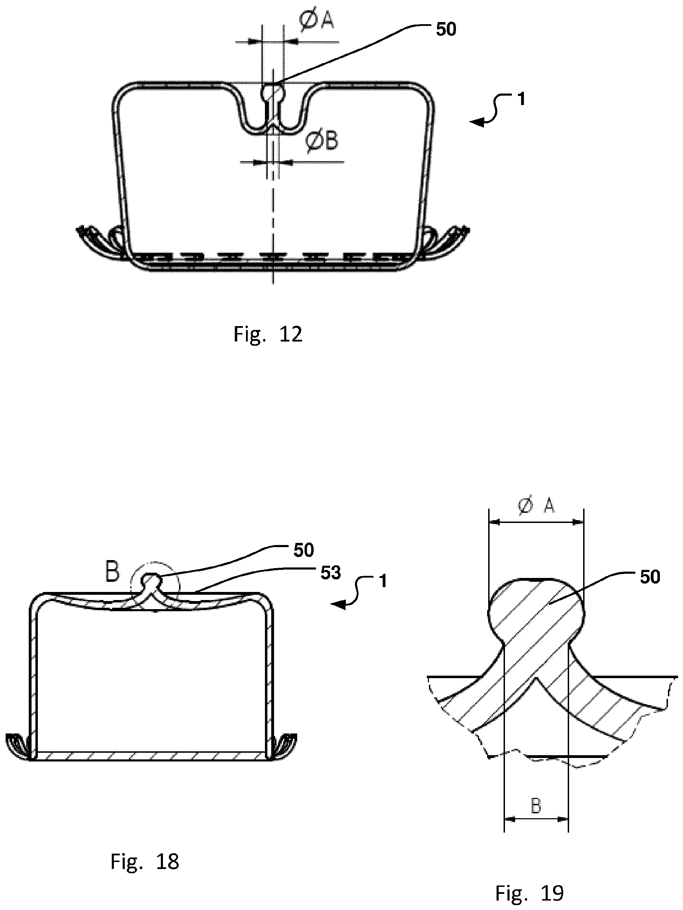

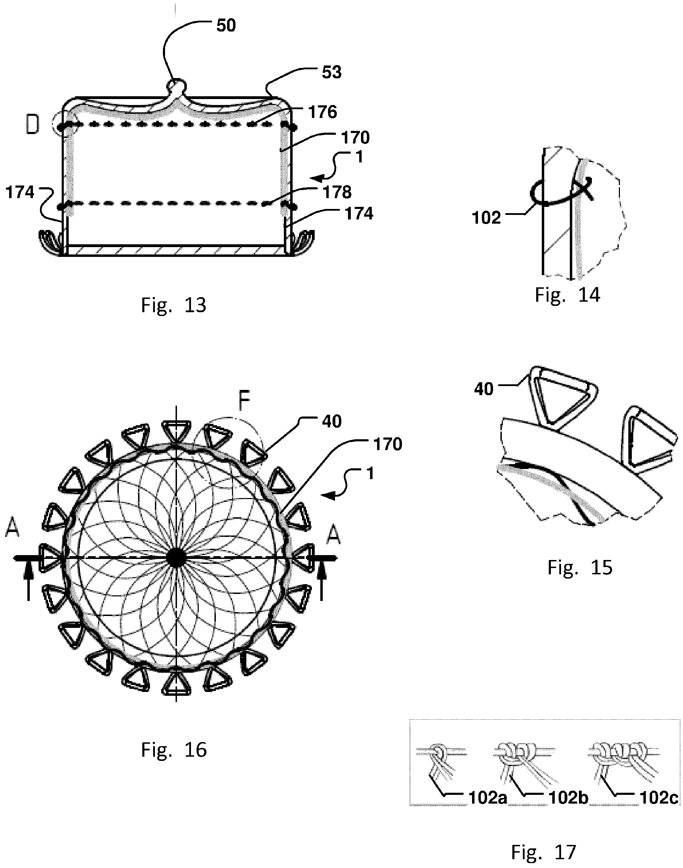

Fig. c1c illustrates forming a loop or knot around the framework. Other examples of knots are illustrated in Figs. 13 and 19. In its pre-configured shape, the anchor strand may be folded. TThe free ends 7a, 7b may be held togetherheld together, as indicated in Fig. 1c. A loop 13may be formed where the anchor strand is folded. The loop 13 may be bent towards the distal end 5 such that it is spaced apart from the proximal end 5 of the anchor member and towards the distal end 6 of the anchor member. Hence, the anchor strand may extend from the first free end 7a to the fold 13 and may return to thesecond free end 7b. In order to attach the anchor member 4 to the framework 2, the freefree ends 7a, 7b maymay be inserted from a first side of the framework 2 through a first opening 3a of the framework 2 to a second side of the framework 2, returned to the first side of the framework 2 via a second opening 3b of the framework 2, and through the loop 13. Hence, when tied, two loops, one by each leg, may form a knot around the framework 2. In order to further attach the anchor member 4 to the framework 2, the free ends 7a, 7b may be inserted from the first side of the framework into an opening 3 of the framework, which may be the second opening 3b of the framework 2, and be interlaced with the framework 2. After being returned to the first side through a third opening 3c, the free ends 7a, 7b may be spaced apart. The free ends may be interlaced with the framework 2 when held together. Inserting the free ends 7a, 7b into the same second opening 3b after forming the knot will push the loop 13 towards the second side of the framework. This in turn will provide a tight knot or loop, such that the loop 13 does not damage surrounding tissue.

IIn the illustrated embodiment, the proximal end 5 of the anchor strand forms two loops or a knot that extend around a portion of the framework 2. AlsoAlso, the loops or knot is formed at an intersection of the framework 2, such as at a crossing of two wires of the framework 2 when formed by wires, such as when the framework 2 is braided. The loops or knot may be formed holding the free ends 7a, 7b together and starting at the proximal end 6 of the anchor strand. In other embodiments, the anchor strand is interlaced with the framework 2 using only one end of the free ends 7a, 7b starting from the distal end 5 of the anchor strand. One free end 7a may be positioned at the distal end 6 of the anchor strand, and the other free end 7b may be interlaced with the framework 2, one or more loops formed around the framework 2. Then,the free end 7b of the anchor strand being interlaced is either cut at the proximal end 5, or again interlaced with the framework to return to the distal end 6 to form the second leg and before ending or being cut at the distal end 6, e.g. such that the free end 7b extends from the framework as illustrated in Fig. d1d.

In the illustrated embodiments, a first loop 14a and a second loop 14b that form two loops extend through the same openings 3a, 3b of the framework 2. Hence, first and second legs of the anchor member 4 extend through the same openings 3a, 3b, 3c of the framework 2. Furthermore, the legs may be located closer together at the proximal end 5 of the anchor member 4 than at the distal end 6 of the anchor member 4, as is illustrated in Figs. 1d. This may be achieved with heat setting the anchor strand such that is has a relaxed configuration where the legs are spaced apart a larger distance at the distal end 6 than at the proximal end 5. Hence the free ends 7a, 7b are spaced apart in the relaxed configuration, such that reliable anchoring into the vascular structure may be achieved. At the same time, the legs may extend through the same openings 3a, 3b, 3c such that the legs receive a slim profile and extend substantially parallel when received into a catheter for delivery to the vascular structure. This avoids damaging the catheter, as discussed above.

As illustrated in Fig. 1d, the anchor strand may comprise a first distal end portion 15a and a second distal end portion 15b. The loop(s) 14a, 14b or knot may be located between the first distal end portion 15a and the second distal end portion 15b. At least one of the first distal end portion 15a, and said second distal end portion 15b may be hook shaped with the free ends 7a, 7b being directed proximally of said implant 1. Hence, the free ends 7a, 7b may be directed proximally towards an attachment member 50 (described below) of the implant 1. The distal end portions 15a, 15b may comprise a bend when the anchor strand returns towards the proximal end from the distal most portion of the anchor member. This may provide a hook shape, which prevents the implant from dislodging after implantation of the implant 1 at the target site.

The anchor member 1, 100 may be monolithic. In the embodiments of Figs. 1a-1d, each anchor member is monolithic, and the implant 1 comprises a plurality of anchor members 4, such as 5-10 anchor members 4 attached to the framework 2.

In the embodiments of Figs. 2a-2d, the anchor member 104 is monolithic and comprises a plurality of interconnected bars 120. At least a portion of the interconnected bars 120 may be interlaced with the framework 102. For example and as is illustrated in Fig. 2c, the interconnected bars may comprise a cross-bar 121, such as located at an intersection or at ends of two interconnected bars 120. The cross bar 121 may extend transverse to the longitudinal axis 110 of the implant 100, such as substantially perpendicularly to the longitudinal axis 110. Hence, the cross-bar 121 may be interlaced with said framework 102 in a radial direction of the implant 100. For example, each end 121a, 121b of the cross bar may be positioned on one side of the framework 102, whereas at least a portion between said ends 121a, 121b is positioned at an opposite side of the framework 102, such as is illustrated in Fig. 2c. The ends 121a, 121b may be arranged substantially flat against the framework 102. In the embodiment of Fig. 2d, the cross-bar forms the anchor member 104, which may extend substantially in the longitudinal direction 110 of the implant when attached to the framework 102. As is illustrated, one end of the cross-bar 121c (which may form the proximal end 105 of the anchor member 104), may be located on one side of the framework 102, whereas a second end, which may form the free end 107, may be located on the opposite side of the framework 102. At least one intermediate section between the ends of the cross-bar, which may form the intermediate sections 108a, 108b discussed above, may be interlaced with the framework 102. Hence, a first intermediate section 108a may be located one side of the framework 102 whereas a second intermediate section 108b may be located on an opposite side of the framework 102.

As is illustrated in Fig. 2b, the interconnected bars 120 may form a circumference of the anchor member 104. The circumference forms a plurality of peaks 122 with a valley 123 between adjacent peaks of the plurality of peaks120. An anchor 102a may be located at a peak and/or a valley of the circumference. At the anchors 102a, such as is illustrated in Fig. 2d, the anchor member 104 may comprise or form a fixation tab. A plurality of fixation tabs may be spaced around the circumference. The fixation tabs may extend in the longitudinal direction of the implant and on opposite sides of said framework, and may be formed as the cross-bar discussed above.

The bars 120 of the framework 102 may extend between the peaks 122 and the valley 123 and on an inner side of the framework 102. At least one of the proximal portion 105 and the distal portion 106 of the anchor member 104 may extend on an exterior side of the framework 102. The proximal portion may extend on the exterior side as is illustrated in Fig. 2d. The distal portion 106 may extend of the exterior side as is illustrated in Fig. 2c. The anchors 102a may be positioned at either a valley 123, as is illustrated in Fig. 2b, or at a peak 122, as is illustrated in Fig. 2d.

The framework 102 illustrated in Figs. 2a-2d may be formed e.g. from a tubular structure of shape memory material, which is cut into the desired shape before being heat treated to the pre-configured shape. Alternatively, separate elements may be welded together, such as from rods of the shape memory material, to a desired shape before being heat treated to the pre-configured shape.

In the embodiments of Figs. 1a-1d and Figs. 2a-2d, the anchor member 4, 104 and the implant 1, 100 are separate components. This facilitates production and makes the implant more flexible. The framework may be used with either multiple anchor members 2, as is illustrated in Fig. 1a, or with a monolithic anchor member 104 comprising a plurality of anchors 102a, as is illustrated in Figs. 2a-2d.

Embodiments may comprise a method of attaching at least one anchor member 4, 104 to an expandable vascular implant 2, 102, such as disclosed above and as disclosed below. The expandable vascular implant comprising the framework 2, 102 may be provided. The anchor member 4, 104 and the framework 2, 102 may be provided as separate components. The anchor member 4, 104 having the proximal portion 5, 105 and the distal portion 6, 106 may also be provided. The distal portion 5, 105 of the anchor member 4, 104 comprises at least one free end 7, 107 configured to extend outwardly from the framework 2, 102. At least a portion of the anchor member 4, 104 may be interlaced with the framework 2, 102, such as between the proximal portion 5, 105 and the distal portion 6, 106. The interlacing may be provided as has been discussed with regard to the various embodiments disclosed above.

The anchor member 4, 104 may be provided in a shape memory material that has been pre-treated to its relaxed pre-configured shape, such as disclosed above. The interlacing with the framework may comprise interlacing the anchor member 4, 104, which is pre-treated before interlacing and made of the shape memory material, with said framework 2, 102. The framework 2, 102 may also be treated to its relaxed shape before the anchor member 4, 104 is attached to the framework 2, 102.

As discussed, the anchor member 4 may be provided as an anchor strand. Interlacing at least the proximal portion 5 of the anchor member 4 with the framework 2 may comprise threading at least a portion the anchor strand through openings 103a, 103b, 103c of the framework and forming at least one loop with the anchor strand around a portion of the framework 2.

The framework 2, 102 may be a tubular braided framework. The braid of the framework 2, 102 may be closed at one end by the braid, i.e. be sack shaped, and by an attachment member at the other end. The attachment member may e.g. be a clamp or a weld, which a delivery wire of the catheter may engage for delivery of the implant 1, 100 to the target site. Alternatively, the braid is tubular and closed at both ends with an attachment member, such as a clamp or weld.

In the embodiments of Figs. 1a-1d, the anchor member 4 may be attached before or after closing the ends of the implant. In the embodiments of Figs. 2a-2d, the anchor member 104 may be introduced into the tubular braid while at least one of the ends of the braid is still open. Hence, one or both ends of the braid may be closed after the anchor member 104 has been inserted into the tubular braid.

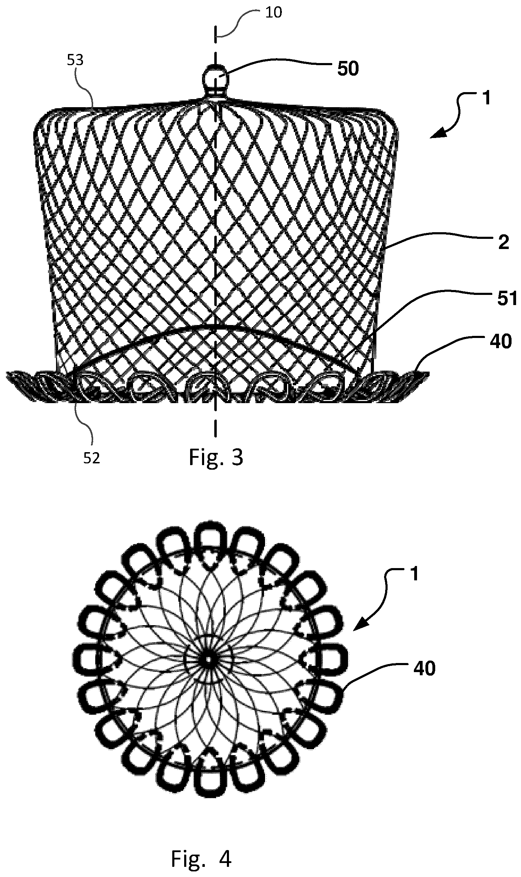

One embodiment of the implant 1, 100 without the anchor member 4, 104 attached is depicted in Fig. 3. Fig. 3 is a side view of an implant 1, such as an LAA occluder. This figure shows an implant 1 comprising strand loops 40 forming the framework 2,102. The strand loops 40 may contribute to preventing the implant 1 from slipping and/or moving from the position once implanted, since the strand loops can fixate the implant to a body wall. These strand loops further enhances the stability of the implant 1, 100 and adds to the stability provided by the anchor member 4, 104. The strand loops 40 may be made from strands. The strands may be made of shape-memory materials, metal, super elastic alloys, Nitinol, or polymers, such as biodegradable polymers. Thus, the strands may be wires. If the strands are made of Nitinol, then the strands may be heat-treated and a very flexible self-expanding wire-mesh can be obtained. The strands may also be formed from a monolithic starting material, such as a tubular member which may be formed into the strands, e.g. by laser cutting.

As can be seen in Fig. 3, strand the loops 40 may be located at one side, such as the distal side, of the implant 1 and a coupling or attachment member 50 on the opposite side, such as the proximal side, of the implant 1, 100. However in embodiments, the strand loops 40 may be positioned on the same side of the implant as the coupling or attachment member 50. The coupling or attachment member may be formed by a laser or plasma weld, which also attaches free ends of a braid that forms the framework 2, 102.

As can be seen in Fig. 3,the strand loops 40 forming part of the framework 2, 102 may have of a shape that extends out of a plane perpendicular to the longitudinal axis of the implant 1, 100. The peripheral edges may be bent out of the perpendicular direction towards an end of the implant 1, 100, such as towards the proximal end of the implant 1, 100. The strand loops 40 may be triangular with rounded corners. However, the shape may round or oval and bent into a desired 3D shape. By shaping the strand loops 40 in this manner, the stabilization of the implant 1, 100 will be further improved, and thus the implant 1, 100 is further prevented from slipping and/or moving from the position once implanted, which contributes to the stability together with the anchor member 4, 104. Hence, the retention and stability may be improved. The bent peripheral edges of the strand loops 40 provide for a defined outwardly oriented spring like force when implanted. The spring force is preferably much lower than the force of the implant for returning to an expanded shape from a collapsed shape. In addition, the loops provide for a controllable spring force irrespective of the main body of the implant 1, 100 being fully expanded.

The spring force of the loops may be provided in a radial direction and an axial direction thanks to the advantageous bending of the peripheral edge. As the number and shape of the loops may be varied, a large flexibility and adaptability to different defects to be occluded is provided in a cautious and reliable manner by embodiments of the device 1.

As can also be seen in Fig. 3, a distal end of the framework 51 may be curved from a circumference of the distal end 52 of the implant 1 towards a centre of the distal end of the framework 51 and towards the proximal end 53 of the implant 1. For example, the distal end of the framework 51 may be at least partly concave in a direction towards the proximal end 53 of the implant 1. Alternatively or additionally, the distal end of the framework 51 may form an angle relative a longitudinal axis of the implant 10 and in a direction towards the proximal end 53 of the implant 1. The curved shape of the distal end of the framework 51 facilitates reduction of the length of the implant 1 when compressed, which enables easier placement and allows for implanting the device deeper into a body cavity, such as the LAA. Furthermore, curved shape of the distal end of the framework 51 increases the stiffness of the implant 1, 100 at the distal end. This gives the hooks more stability and prevents the rotation of the implant 1, 100 when implanted.

Fig. 4 is a top view of an implant 1, 100 without the anchor members 4, 104 attached. In this embodiment the strand loops 40 are of a round shape. By giving the strand loops 40 a round shape, the strand loops 40 are less prone to break. Hence, together with the anchor member 4, 104 when attached, this also contributes to the reliability of the implant 1, 100. The embodiment of Fig. 4 may be combined with the embodiment of Fig. 3, such as the shape of the distal end of the framework 51.

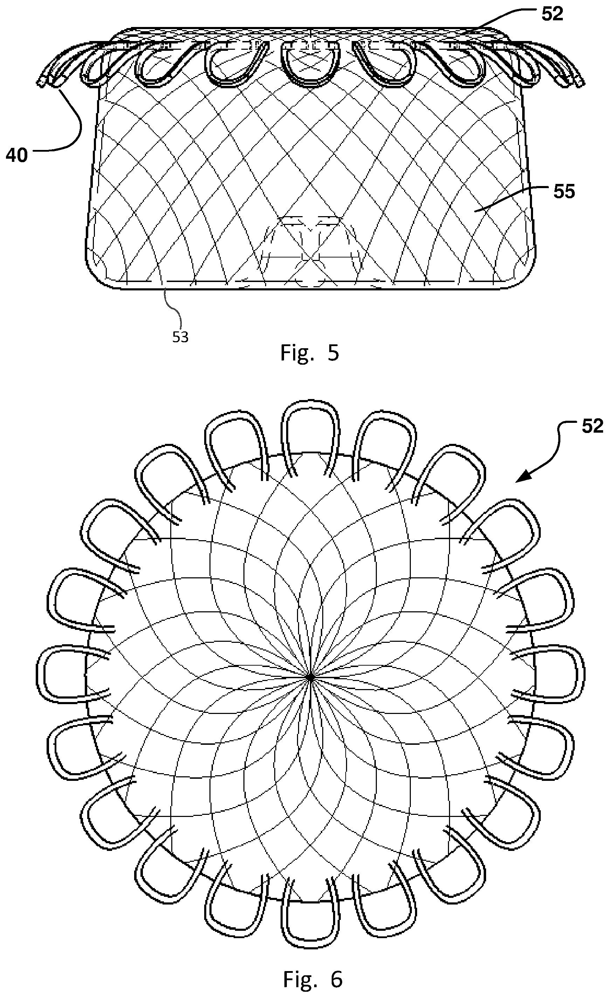

Fig. 5 is a side view of an implant, in this case an LAA occluder, showing another embodiment 1, 100 without the anchor members 4, 104 attached. A longitudinal section 55 of the framework is in this figure shaped as a frustum of a hollow cone-shaped cylinder. The strand loops 40 surround the rim of the hollow cone-shaped cylinder and are extendable outwardly from the hollow cone-shaped cylinder substantially perpendicularly to a centre axis of the hollow cone-shaped cylinder. The cone shape provides for a reliable positioning avoiding embolization of the device in a defect, such as the LAA. The principle may be compared to a cork that has a larger diameter at one end. The larger diameter of the implant shown in Fig. 4 is upon implantation positioned at or towards the proximal end. Hence, this together with the anchor member 4, 104 contributes to the stability and reliability of the implant 1, 100. The embodiment of Fig. 4 may be combined with the embodiment of Fig. 3, such as the shape of the distal end of the framework 51.

The implant 1, 100 may also be of another shape, such as cylindrical. **It** can comprise different sections, some of which may be cone-shaped and some of which may be cylindrical.

Fig. 6 is a top view of an implant 1, 100 without the anchor members 4, 104 attached. In one embodiment, shown in Fig. 7, half of the strands, which will also be used for the longitudinal section 55, form a cover for the implant on the distal end 52. The remaining strands, which will also be used for the longitudinal section 55, project outwards on the edge of the cover as strand loops 40. Thus, on the distal side 172, strands which form the strand loops 40 are not integrated in the braid. On a proximal end 53, opposite to the distal end 52, as well as along the longitudinal section 82, all strands from the outer edge up to the rotation axis are braided or intertwined. Thus, the configuration or distribution of strands to strand loops can be said to be 2:1. However, it is also possible to have other distributions, such as 1:1, 1:2, 1:3, 3:1 etc. **It** is also possible to make the implant, which has only strand loops on the proximal side 172, i.e. forming an open mesh or a round longitudinal braid, closed on one side only. The embodiment of Fig. 4 may be combined with the embodiment of Fig. 3, such as the shape of the distal end of the framework 51.

Fig. 7 is a cross-sectional view of an implant with a membrane 200 and without the anchor member 4, 104 attached. The membrane 200 is also illustrated in Figs. 1a and 2a. The membrane 200 may be attached to the implant 1, 100 with a strand or thread 202. In this embodiment, the membrane 200 is attached to the outer surface of the implant 1, 100, on the same side as the coupling 50. The membrane 200 can cover the whole circumference of the implant 1, 100 and is then also stitched to the implant 1 with a seam 204. The use of membranes or inner membranes results in improved occlusion and rapid endothelialisation. The use of a membrane 200 also results in an ideal closure of e.g. the left atrial appendage, since it seals the gap instantly. In other embodiments, the membrane 200 may cover only a portion of circumference of the framework, such as illustrated in Figs. 1a, and 2a. The membrane 200 may cover the proximal portion of the anchor member 4, 104. Hence, any part of the anchor member 4, 104 may be covered by the membrane 200, e.g. to protect the catheter or vascular tissue from damage during delivery to the target site. Yet, the legs of the anchor member 4, 104 may be exposed such that they may be collapsed during delivery of the implant.

Fig. 8 is an enlarged sectional view of a thread used for attaching a membrane 200 to the implant 1, 100. The thread 202 is wound around at least one strand of the implant.

Fig. 9 is a top view of an implant 1, 100 with strand loops 40. The strand loops 40 are located all around the circumference of the implant 1, 100. Also, the membrane 200 can be seen in Fig. 9. Here the membrane 200 covers the whole circumference of the implant 1.

Fig. 10 shows the strand loops 40 of the implant 1 in more detail and without the anchor member 4, 104 attached. The strand loops 40 in Fig. 10 have a rounded shape, i.e. a somewhat rectangular shape with rounded corners. Rounded corners are tissue friendly avoiding deep tissue penetration and possible ruptures or leakages.

Fig. 11 is a view of different knots used for attaching the membrane to the implant 1, 100. These knots may also be used to attach the anchor member 4 to the framework 2. The thread 202 can be secured to the framework 2, 102 with a single knot or with a double knot. The ends of the thread 202 can be thermally treated to provide further reliability of the fixation.

Fig. 12 is cross-sectional view of an implant 1, 100 without the anchor member 4, 104 attached and with a coupling or attachment member 50. The coupling or attachment member 50 may be formed into the shape of a ball pivot. The ball pivot can be connected to a flexible pusher of a delivery device, which can be used to move the implant in a sheath of a catheter, e.g. for delivery of the implant 1, 100 and/or retrieval of the implant 1, 100 prior to being decoupled. The coupling or attachment member 50 is in this embodiment sunk or lowered into the implant 1 and will not impede blood flow at the target site, e.g. in a body vessel, where it is situated after having been delivered. In this embodiment, the framework 2, 102 of the implant 1, 100 has been formed with a hollow space for the coupling or attachment member 50. Hence, the coupling or attachment member 50 may be located at least partially distal of a top or proximal surface of the implant. The top surface may form the proximal most portion of the implant. The lowered coupling or attachment member 50 provides for a shorter overall length compared to positioning the coupling or attachment member 50 at the top surface. This may prevent dislodging the implant after implantation and provides for reliable anchoring of the implant 1, 100 together with the anchoring member 4, 104. The coupling or attachment member 50 will therefore not impede blood flow that may dislodge the implant 1, 100. In another embodiment according to Fig. 13, the proximal end or surface 53 is instead given a concave or curved shape to position the coupling or attachment member 50 at least partially distal of the proximal most part of the implant 1, 100. With the coupling or attachment member 50, the flexibility during delivery is increased, since the implant 1, 100 is retrievable.

In the embodiment depicted in Fig. 13, the implant 1, 100 has a membrane. This membrane may also be provided with the embodiments of Figs. 1a-1d, and Figs. 2a-2d. The membrane is an inner membrane 170. The inner membrane 170 may be a special thermo-treated PET-knit fabric. The inner membrane 170 may be attached to the inner surface of the framework 2, 102, such as after attachment of the anchoring member 4, 104 to the framework 2, 120. The inner membrane 170 may be attached to the implant 1 with a strand or thread. In this embodiment, the inner membrane 170 is attached to the inner surface of the implant 1, on the same end of the implant 1, 100 as the coupling or attachment member 50, i.e. the proximal end 53. The inner membrane 170 may extend in a longitudinal direction at least partially along the longitudinal sides 174 of the implant 1, 100. The inner membrane 170 can cover the whole circumference of the implant 1, 100 and may be also stitched to the implant with seams 176, 178. It may also only cover the proximal portion 5, 105 of the anchor member 4, 104, whereas the distal portion 6, 106 is exposed, i.e. not covered by the inner membrane 170. The use of a membrane 200 and/or inner membrane 170 may provide for improved occlusion and rapid endothelialisation.

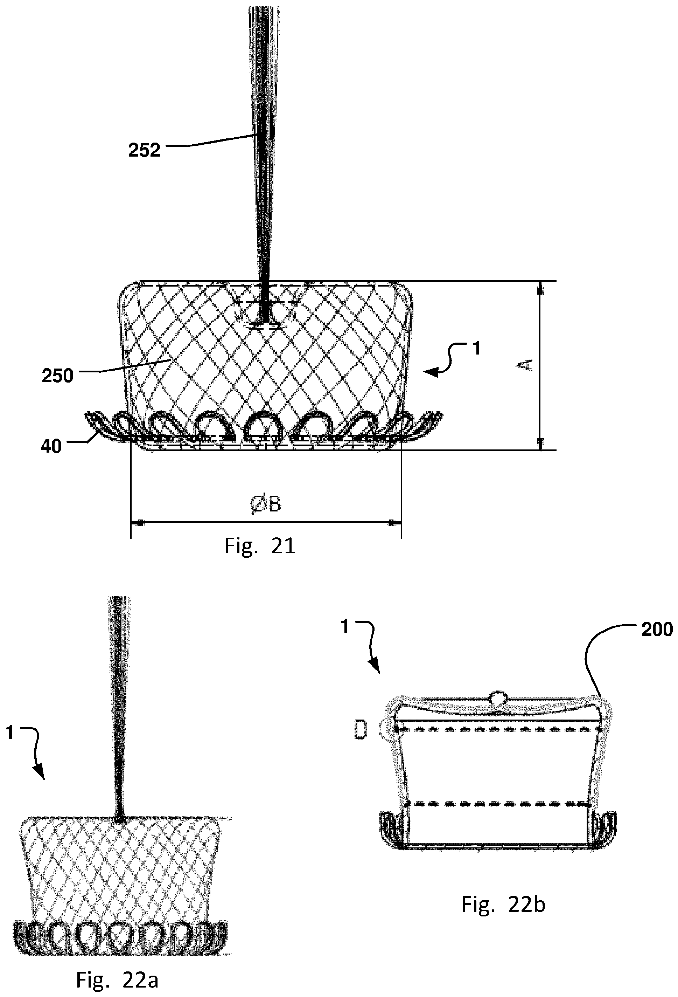

In another embodiment depicted in Fig. 22b, an outer membrane 300 is attached outside the implant 1, 100 in a similar manner as the inner membrane 170. Also the outer membrane 300 may be a special thermo-treated PET-knit fabric. In one embodiment, the implant 1, 100 is covered with membranes 170, 200 both on the inside and the outside of the framework 2, 102. As an alternative of using a membrane 170, 200, the implant 1, 100 may instead be covered or coated using polymer spinning technology, such as solution blow spinning or electrospinning, or a dipping method. The coating as well as the membrane 170, 200 may be made of a biocompatible and implantable material, such as PTE, PTFE or PUR. The inner and/or outer membrane 170, 200 or coating may be provided as a fibrous or non-fibrous film membrane that may have an initial controllable fluid retention by perforations or microperforations thereof. The membrane may cover the entire expanded diameter of the implant. Alternatively, it may only cover portions thereof, such as illustrated in Figs. 1a, and 2a. The portions may be as small as the cell structure of the fabric of the implant 1. For instance one or more cells of a braiding may be provided with a coating extending the space between adjacent strand portions forming the cells. The coating may cover the proximal portion 5, 105 of the anchor member 4, 104, as is illustrated in Figs. 1a and 2a. Hence, the distal portion 6, 106 of the anchor member 4, 104 may be exposed, i.e. not covered by the coating.

In this manner, different perfusion rates may be adjusted to different areas of the device. It may for instance be desired to obtain an inflow of blood into the inner of the expanded implant 1, 100 from a distal end thereof to enhance integration of the implant 1, 100 with surrounding blood upon clotting thereof. A reduced or prohibited outflow of blood through the proximal end may however be provided by a tighter membrane or larger diameter/surface/cells of the device being covered than those of another section of the implant 1, 100.

The coating or membrane 170, 200, 300 may be affixed to the implant 1, 100 in its expanded shape. In this manner, the coating or membrane is free of tension which advantageously avoids pre-mature fatigue thereof allowing for a reliable ingrowth.

The coating or external membrane may alternatively be affixed to the implant 1, 100 in its collapsed shape.

Patterns of covered cells may be provided to efficiently control a desired flow pattern upon implantation. In this manner, the occlusion is not abrupt upon implantation. A certain blood flow may still occur after implantation and gradually decline upon blood coagulation and/or endotheliazation of the implanted device.

**It** should be noted that the aforementioned principles of coatings/membranes may be provided with other implants than the examples shown herein, e.g. ASD, PFO, PLD or VSD occluders, stents or other implants for a vascular structure.

Fig. 14 is an enlarged sectional view of a thread 102 used for attaching a membrane to an implant 1, 100. The thread 102 is wound around the framework 2, 102, such as at least one strand thereof, for attaching the membrane to the implant 1, 100.

Fig. 15 is an enlarged sectional view of strand loops 40 of the implant 1, 100 of Fig. 16. The view in Fig. 15 corresponds to section F in Fig. 16.

Fig. 16 is a top view of an implant 1, 100 shown without the anchor member 4, 104 attached. The triangular strand loops 40 are located all along the perimeter of the implant 1, 100. Also in Fig. 16, the inner membrane 170 can be seen.

Fig. 17 is a view of different knots 102a, 102b, 102c for the implant. The thread 102 can be secured to the framework 2, 102, such as a strand thereof, of the implant 1, 201 with a loop 102a around a portion of the framework, a single knot 102b or with a double knot 103c. The ends of the thread 102 can be thermally treated.

Fig. 18 is a lateral view of an implant with a coupling 50 or attachment member and without the anchor member 4, 104 attached to the implant 1, 100. As can be seen from this figure, the braiding of the implant 1, 100 is at the proximal end 53 of the implant 1, 100 formed so that the proximal end 53 can be positioned towards the distal end 52 of the implant 1, 100 compared to the proximal most portion of the implant 1, 100.

Thus, the coupling or attachment member 50 extends less from proximal most portion of the implant 1, 100 and will impede the blood flow, such as in the atrium when the implant is an LAA implant, to a lower extent at the target site when the proximal end 53 faces the atrium of the heart.

Fig. 19 is an enlarged sectional view of a coupling or attachment member 50 of the implant 1, 100. The coupling or attachment member 50 may be formed by welding a portion of the framework 2, 102, such as strand ends, together after the framework has been formed, such as by a braiding machine has finished the braiding or intertwining of the implant 1, 100. The coupling 50 or attachment member may be formed by welding it into a ball pivot. The strands are merging into the welded clot in a not-straight, i.e. not parallel manner in the example shown in Fig. 19. The ball pivot can be connected to a socket of the flexible pusher, which can be used to move the implant 1, 100 in a sheath of a catheter for delivery. The pusher may be able to rotate the implant 360 degrees, when the ball pivot is connected to the socket. The framework 2, 102 of the implant 1, 100 can be compressed so that the implant can be inserted into the sheath. After leaving the sheath, the implant 1, 100 independently reassume the predetermined shape and ensure an interlocking hold.

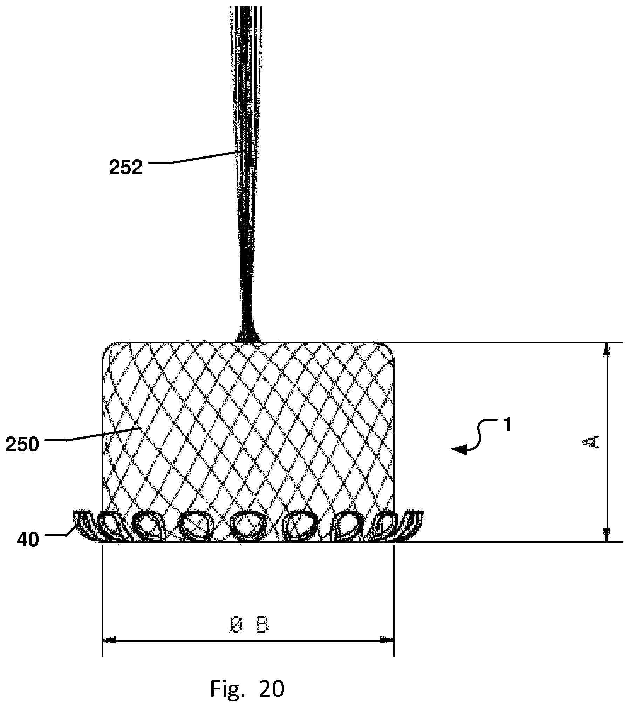

Fig. 20 is a side view of an implant 1, 100 being manufactured and before the anchor member 4, 104 has been attached to the framework 2, 102. This figure shows the implant 1, 100 after the framework has been formed. In the figure, the framework is illustrated as formed by strands 252. These strands 252 are cut to an appropriate length and may be welded together to form the coupling or attachment member 50 shown in, e.g., Figs. 18-19.

Fig. 21 is a side view of an implant 1, 100 being manufactured and before the anchor member 4, 104 has been attached to the framework 2, 102. This figure illustrates the implant 1, 100 after the framework 2, 102 has been formed. In the figure, the strands 252 are shown as forming the framework 2, 102. The framework 2, 102 of the implant 1, 100 is positioned at least partially distal of the proximal end of the implant 1, 100 to accommodate a hollow space for the coupling or anchor member 50. The strands 252 may be cut to an appropriate length and welded together to form the coupling 50 or attachment member shown in, e.g., Fig. 14.

An implant 1 with a conical shape can be seen in Fig. 22a. Due to the conical shape of the implant, higher radial forces from the body walls are possible. Therefore, the risk of perforation is lowered.

Other possible shapes of the occluder are elongated, round, cylindrical, flat or dumbbell-shaped.

Although, the strand loops 40 are depicted in the figures as situated in one row i.e. are axially aligned along the longitudinal axis. However, it is possible to have strand loops 40 in multiple rows, e.g. two rows, axially spaced apart along the longitudinal axis 10, 110 of the implant 1, 100.

The strand loops 40 may be situated either on the proximal end 53 or the opposite side, i.e. the distal end 52. **It** is further possible to have strand loops 40 on both the proximal end 53 and the distal end. This results in a fixation of the implant in both directions, and contributes to stability together with the anchoring member 4, 104.

In an embodiment the implant 1, 100 may be provided with a coating. The coating may be applied to an external surface of the implant 1, 100. The implant may be coated on the outside by a method, in which the implant 1, 100 is dipped in a solution with a specific viscosity, while the implant 1 is in an expanded shape. By applying the coating to the implant 1, 100 while is in an expanded shape, the coating will be free of tension, which advantageously avoids pre-mature fatigue thereof and thus allows a reliable ingrowth. Application of a coating to the implant 1, 100 while the implant 1, 100 is in an expanded shape, may also be advantageous for other reasons, such as the fact that the implant 1, 100 can be made very flexible and that a particularly large expansion/contraction ratio, i.e. a ratio of a size or diameter of the implant 1, 100 in its expanded shape and the size or diameter of the implant 1, 100 in its contracted shape, can be obtained for the implant 1, 100.

In another embodiment, the end and part of or the whole side are dipped into the solution, so as to be provided with coating, such as illustrated in Figs. 1a, and 2a such that the anchoring member 4, 104 is partially covered with the coating. Thereby, a large portion of the implant 1 is covered with the coating. In yet another embodiment, only the ends of the implant 1 are dipped into the solution, wherein the anchoring member is exposed. However, the implant may be covered with the coating at both ends. This can be done by first dipping the end into the solution, then retracting the implant 1 from the solution. Thereafter, the implant is turned around and with the other end facing the solution, the device is again dipped into the solution. A coating applied to the implant 1, 100 provides for an improved occlusion, improved sealing of a defect, such as a heart defect, an improved endothelialization and/or for slowing down the blood flow through the defect.

According to an embodiment, in order to provide some body liquid to pass through the implant 1 once implanted, the coating may be provided with perforations or microperforations. Thus, by the use of perforations or microperforations, the occlusion is not abrupt, but formed gradually over time.

The membrane 200 or coating may comprise a polymer, such as polyurethane, polytetrafluoroethylene (PTFE) or expanded polytetrafluoroethylene (ePTFE). The use of e.g. PTFE or ePTFE may provide for low friction. The polymer may be applied to the implant 1, 100 by e.g. dipping, spraying, such as electro-spraying, or polymer spinning technology, such as electro-spinning or solution blow spinning. For example, the membrane may be formed by a fibrous polymeric covering, e.g. formed by applying the polymer as indicated. The membrane 200, e.g. the fibrous polymeric covering, may at least partially cover the framework 2, 102, as is illustrated in Figs. 1a and 2a and discussed above. Additionally or alternatively, the membrane 200, e.g. the fibrous polymeric covering, may at least partially cover at least one anchor member 4, as is illustrated in Fig. 1a and discussed above. As another alternative, instead of coating the implant 1, 100 a non-fibrous film membrane may be sewed onto the external surface of the implant 1, 100. Such a non-fibrous film membrane may be manufactured by applying a film on a substrate and then removing the substrate. E.g. a film can be applied by dipping a substrate into a solution and thereafter removing the substrate. As an alternative, a substrate can be sprayed and thereafter removed from the coating formed by the spray, so that a non-fibrous film membrane is obtained.

The embodiments presented in Figs. 4-22b may be combined with each of the embodiments presented with regard to Figs. 1a-2d. Furthermore, such combinations may also be combined with the embodiments presented with regard to Fig. 3, and particularly with regard to the shape of the distal end of the framework 52.

**It** should also be appreciated that features disclosed in the foregoing description, and/or in the foregoing drawings and/or following claims both separately and in any combination thereof, be material for realizing the present invention in diverse forms thereof. When used in the following claims, the terms "comprise", "include", "have" and their conjugates mean, "including but not limited to".

The present invention has been described above with reference to specific embodiments. However, other embodiments than the above described are equally possible within the scope of the invention. Different method steps than those described above may be provided within the scope of the invention. The different features and steps of the invention may be combined in other combinations than those described. The scope of the invention is only limited by the appended patent claims.

### Examples of the disclosure include the following examples:

1. An expandable vascular implant, comprising
   a framework with openings and forming an exterior surface of said implant;
   at least one anchor member for anchoring the implant to a vascular structure and having a proximal portion and a distal portion; wherein
   the distal portion of the anchor member comprises at least one free end configured to extend outwardly from said framework; and
   the anchor member is at least partly interlaced with said framework between said proximal portion and said distal portion such that said anchor member is fixed to said framework.
2. The expandable vascular implant of any of the previous example, wherein said anchor member comprises an intermediate section between said proximal portion and said distal portion, wherein said intermediate section is interlaced with said framework.
3. The expandable vascular implant according to example 2, wherein the intermediate section is curved, and a proximal part of the intermediate section is configured to extend along a first side of said framework, and a distal part of said intermediate section is configured to extend along a second side of said framework, said first side and said second side of said framework being opposite sides of said framework.
4. The expandable vascular implant according to any of examples 1-3, wherein said anchor member comprises an anchor strand formed into said anchor member, wherein a proximal end of said anchor strand forms at least one loop extending around a portion of said framework, and wherein said anchor strand comprises two free ends forming a distal end of said anchor member, said loop being positioned between said two free ends.
5. The expandable vascular implant of example 4, wherein said proximal end of said anchor strand forms two loops extending around said portion of said framework.
6. The expandable vascular implant of example 5, wherein a first loop and a second loop of said two loops extend through the same openings of said framework.
7. The expandable vascular implant of any of examples 4-6, wherein said anchor strand comprises a first distal end portion and a second distal end portion, and said at least one loop is located between said first distal end portion and said second distal end portion, wherein at least one of said first distal end portion and said second distal end portion is hook shaped with said free ends being directed proximally of said implant.
8. The expandable vascular implant according to any of examples 1-3, wherein said anchor member is monolithic and comprises a plurality of interconnected bars, wherein at least one of said plurality of bars comprises a cross-bar, wherein said cross-bar is interlaced with said framework in a radial direction of said implant.
9. The expandable vascular implant according to any of examples 1 or 8, wherein the interconnected bars form a circumference of said anchor member, and wherein the circumference forms a plurality of peaks with a valley between adjacent peaks of said plurality of peaks, and wherein an anchor is located at a peak and/or a valley of said circumference.
10. The expandable vascular implant according to example 9, wherein the anchor member comprises a plurality of fixation tabs spaced around said circumference, said fixation tabs extending in the longitudinal direction of the implant and on opposite sides of said framework.
11. The expandable vascular implant according to any of examples 9-10, wherein bars of said anchor member extend between said peaks and said valley and on an inner side of said framework, and wherein at least one of the proximal portion and the distal portion of said anchor member extend from at least one of said peak and valley and on an exterior side of said framework.
12. The expandable vascular implant according to any of the previous examples, wherein anchor member and the implant are separate components.
13. A method of attaching at least one anchor member to an expandable vascular implant, comprising
   providing an expandable vascular implant comprising a framework;
   providing at least one anchor member having a proximal portion and a distal portion, wherein the distal portion of the anchor member comprises at least one free end configured to extend outwardly from said framework; and
   interlacing at least a portion of the anchor member between said proximal portion and said distal portion with said framework.
14. The method according to example 13, wherein providing said anchor member comprises providing said anchor member in a shape memory material that has been pre-treated to its relaxed shape, and wherein said interlacing with said framework comprises interlacing said anchor member, made of the pre-treated shape memory material, with said framework.
15. The method according to example 13 or 14, wherein providing said anchor member comprises providing said anchor member as an anchor strand, and wherein interlacing at least the portion of the anchor member with said framework comprises interlacing at least a portion said anchor strand and forming at least one loop with said anchor strand around a portion of said framework.

## Claims

1. An expandable an collapsible LAA occluder, comprising
a braided framework with openings and forming an exterior surface of said LAA occluder;
at least one anchor member for anchoring the implant to a LAA structure and having a proximal portion and a distal portion; wherein
the distal portion of the anchor member comprises at least one free end configured to extend outwardly from said framework; and
the anchor member is at least partly interlaced with said framework between said proximal portion and said distal portion such that said anchor member is fixed to said framework; and said LAA occluder comprises at least one conical shape section.

2. The occluder of claim 1, comprising multiple of said anchor members.

3. The occluder of claim 1 or 2, comprising a membrane attached to the implant.

4. The occluder of claim 3, wherein the membrane is attached to said implant with at least one knot and said anchor member is also attached to the framework with said knot.

5. The occluder of any of claims 1 to 4, wherein one or more cells of the braided framework are provided with a coating extending the space between adjacent strand portions forming the cells, and wherein said coating covers a proximal portion of the anchor member and a distal portion of the anchor member is not covered by the coating.

6. The occluder of any of claims 1-5, comprising an inner and/or outer membrane or a coating having a controllable fluid retention by perforations thereof.

7. The occluder of any of claims 1-6, wherein said anchor member and the occluder are separate components.

8. The occluder of any of claims 1-7, wherein said framework includes cells and said cells are covered in a pattern.

9. The occluder of any of claims 1-8, wherein said framework is closed at one end or both ends of the occluder and said anchor member is fixed to said framework before closing one or more ends of the occluder.

10. The occluder of any of claims 1-9, wherein said framework is a tubular braid.

11. The occluder of any of claims 1-10, wherein said anchor member is a hook.

12. The occluder of claim 11, wherein said hook comprises a barb.
